# EUROPEAN PATENT APPLICATION

(11) **EP 4 082 584 A1**
(43) Date of publication of application: **02.11.2022**
(21) Application number: 20906332.0
(22) Date of filing: 15.12.2020
(51) Int. Cl.: A61L 2/20, A61L 9/00, A61L 9/20

(54) **GAS TREATMENT METHOD, STORAGE CHAMBER, INCUBATOR, AND STERILIZER**

(30) Priority: 25.12.2019 JP 2019234723
(71) Applicant: Ushio Denki Kabushiki Kaisha, Tokyo 100-8150 (JP)
(72) Inventor: NAITO,Keisuke, Tokyo 100-8150 (JP)
(74) Representative: Tomerius, Isabel
(86) International application number: PCT/JP2020/046696
(87) International publication number: WO 2021/131888

(57) **Abstract**

A gas treatment method is provided to enable treatment of VOCs with higher decomposition efficiency than before in cases where a gas containing a treatment target substance classified as a VOC exists inside a closed space. The method includes the steps of: (a) drawing a gas inside a closed space into a gas treating apparatus including an excimer lamp; (b) producing radicals and decomposing a treatment target substance by irradiating the gas inside the gas treating apparatus with vacuum UV light from the excimer lamp; and (c) discharging the gas after a treatment and subjected to the step (b) via an ozone decomposition filter into the closed space.

## Description

### TECHNICAL FIELD

The present invention relates to a gas treatment method, and more particularly to a method for treating a gas that exists inside a closed space. The present invention also relates to a storage chamber, an incubator, and a sterilizer that use this method.

### BACKGROUND ART

Medical tools require to be stored always in a sterile condition. A structure disclosed in Patent Document 1 below, for example, is known for storage rooms (storage chambers) for storing such medical tools or instruments.

The storage chamber disclosed in Patent Document 1 includes an air circulation path for forced circulation of air inside the storage chamber. In the air circulation path are disposed a UV lamp for sterilization, and a photocatalyst plate for photocatalytic reactions that enable the decomposition of acetaldehyde or formaldehyde.

### PRIOR ART DOCUMENT

Patent Document 1: JP-A-2006-333954

### SUMMARY OF THE INVENTION

### PROBLEMS TO BE SOLVED BY THE INVENTION

Sterilization methods for medical tools are internationally standardized. Sterilization methods are roughly classified into heat processes and application of chemicals used for sterilization such as formaldehyde, orthophthalaldehyde, glutaraldehyde, EOG (Ethylene Oxide Gas), hydrogen peroxide, or peracetic acid. The heating process is applicable only to medical tools that can be heated. If a medical tool includes a resin part such as fiber parts of an endoscope, for example, thermal sterilization is not applicable because the fiber parts will deform by heat. In such cases, normally, methods that use sterilizing chemicals are employed.

Of the sterilizing chemicals listed above, hydrogen peroxide is applicable to a very limited range of sterilization targets because hydrogen peroxide may cause oxidation of the target objects. For this reason, chemicals such as formaldehyde and ethylene oxide are commonly used for sterilization. Formaldehyde, however, is likely to cause cancer, and therefore EOG is recommended over formaldehyde in recent years (see 'Guideline for Sterility Assurance in Healthcare Setting 2015' edited by Japanese Society of Medical Instrumentation).

Specifically, after performing a sterilization process to a medical tool in which EOG is introduced into a sterilizer, the sterilizer is exhausted of EOG by aeration, to remove EOG adhered to the medical tool. After that, the medical tool that has been sterilized is stored in a storage chamber.

However, ethylene oxide tends to be persistent and it is very difficult to prevent a small amount of ethylene oxide from being left in or on the medical tools. Consequently, the remaining ethylene oxide vaporizes and is released as EOG into the storage chamber during storage inside the storage chamber. There is thus a risk that an operator who opens the door of the storage chamber to take out a medical tool from the storage chamber may inhale EOG inside the storage chamber. While considered to be less carcinogenic than formaldehyde, ethylene oxide is still regarded as a substance hazardous to the human body.

In the storage chamber described in Patent Document 1, a UV lamp is provided for the sterilization of the storage target itself as well as for causing photocatalytic reactions. While Patent Document 1 does not mention ethylene oxide, the document describes the decomposition of acetaldehyde and formaldehyde by photocatalytic reactions. Acetaldehyde and formaldehyde are highly volatile similar to ethylene oxide and classified as VOCs (Volatile Organic Compounds).

The decomposition efficiency of VOCs in a process using photocatalysis as in Patent Document 1 is very poor. This is because the target VOCs contained in the gas decompose only when the gas contacts the photocatalyst, and the VOCs in the gas that flows through a space between the UV lamp and the photocatalyst do not decompose.

Given the issues described above, an object of the present invention is to provide a gas treatment method that enables treatment of VOCs with higher decomposition efficiency than before in cases where a gas containing treatment target substances classified as VOCs exists inside a closed space. Another object of the present invention is to provide a storage chamber, an incubator, and a sterilizer which are, by the use of this gas treatment method, capable of decomposing treatment target substances classified as VOCs contained in the gas inside the closed space with high efficiency.

### MEANS FOR SOLVING THE PROBLEMS

The present invention resides in a gas treatment method for treating a gas inside a space, the gas being an air containing a treatment target substance classified as a VOC and existing inside a closed space, the method including the steps of:
(a) drawing the gas inside the closed space into a gas treating apparatus including an excimer lamp capable of emitting vacuum UV light;
(b) producing radicals and decomposing the treatment target substance by irradiating the gas inside the gas treating apparatus with the vacuum UV light from the excimer lamp; and
(c) discharging the gas after a treatment subjected to the step (b) via an ozone decomposition filter into the closed space.

According to the above method, radicals that are generated by irradiating a gas inside a closed space with vacuum UV light decompose a treatment target substance. Unlike conventional methods that decompose VOCs using photocatalytic reactions, there is no need to bring the gas into contact with a specific location for the decomposition. Consequently, the VOC decomposition efficiency is improved as compared to conventional configurations.

Irradiation of water and oxygen contained in the gas inside the closed space in the above step (b) with vacuum UV light induces reactions expressed by following Formulas (1) and (2). In the following formulas, O(¹D) represents oxygen atoms in the excited state, and O(³P) represents oxygen atoms in the ground state. hv(λ) indicates absorption of light of a wavelength λ.

O₂+ hv(λ) → O(¹D) + O(³P) ···· (1)

O(¹D) + H₂O → • OH + • OH ···· (2)

The treatment target substance decomposes highly efficiently due to the very high reactivity of hydroxyl radicals (• OH) obtained by Formula (2).

Oxygen atoms O(³P) generated by the reaction of Formula (1) react with the oxygen (O₂) contained in the air inside the closed space and generate ozone (O₃) by the reaction expressed by following Formula (6).

O(³P) + O₂ → O₃ ···· (6)

In a case where the above method is applied to a storage chamber for storing medical tools, for example, ozone making contact with the medical tools may deteriorate the medical tools. With this in view, in the step (c), the gas after the treatment is discharged through an ozone decomposition filter into the closed space. Namely, according to the above method, treatment target substances classified as VOCs contained in the gas inside the closed space are decomposed by the step (b), and further, the step (c) decomposes ozone which is a secondary product generated by execution of the step (b).

The ozone decomposition filter may be disposed of such as to allow the gas discharged from the gas treating apparatus to flow through. For example, the ozone decomposition filter may be disposed of inside the gas treating apparatus.

More specifically, the gas treating apparatus may include:
a housing accommodating the excimer lamp;
a gas inlet allowing the gas to be introduced into the housing;
a gas outlet allowing the gas that has been irradiated with the vacuum UV light emitted from the excimer lamp to be discharged out of the housing into the closed space; and
the ozone decomposition filter disposed of inside the housing between the excimer lamp and the gas outlet.

The housing may have a smaller cross-sectional area of a flow path in a space irradiated with the vacuum UV light than a cross-sectional area of an opening at the gas inlet.

Vacuum UV light is strongly absorbed by oxygen. Therefore, if the cross-sectional area of an opening in the space irradiated with vacuum UV light, i.e., the diameter of the gas flow path, is too large, an increased amount of gas may be discharged out of the housing without being irradiated with vacuum UV light, which may reduce the speed at which the treatment target substance decomposes. As described above, the steps (a) and (b) are performed by the gas treating apparatus having a housing in which the cross-sectional area of the opening in the space irradiated with vacuum UV light is made smaller than the cross-sectional area of the opening at the gas inlet. This increases the ratio of the gas irradiated with vacuum UV light to the gas introduced into the housing. This allows an increased amount of radicals to be generated and enhances the efficiency of decomposition of treatment target substances in the gas.

The gas treating apparatus may include a fan, and a power supply unit that drives the fan and the excimer lamp.

The step (a) may be a step of drawing the gas inside the closed space into the gas treating apparatus by the fan, and
the steps (a) to (c) may be repeatedly performed in which the gas discharged from the gas treating apparatus into the closed space by the step (c) is taken into the closed space again by the step (a).

According to the above method, the gas inside the closed space is repeatedly irradiated with UV light from the gas treating apparatus, so that the treatment target substances contained in the gas inside the closed space decompose with even higher efficiency.

The treatment target substance may be a substance containing at least one of the groups consisting of ethylene oxide and aldehydes.

The above substances are all classified as VOCs and are considered to have a significant effect on the human body. Even if gas containing these substances exists inside the closed space, decomposition processes by radicals can greatly reduce the concentration of these substances.

'Aldehydes' herein refer to substances that include an aldehyde group (also called formyl group) such as formaldehyde, acetaldehyde, phthalaldehyde, and glutaraldehyde.

The excimer lamp may include a light-emitting tube enclosed with a light-emitting gas containing Xe. In this case, the excimer lamp emits vacuum UV light having a main wavelength of 172 nm.

The above gas treating apparatus may be placed at an installation position inside the closed space, and
the steps (a) to (c) may be performed while the installation position of the gas treating apparatus inside the closed space is changed.

According to the above configuration, the gas treating apparatus may be moved inside the closed space while the steps (a) to (c) are performed, so that, even when the gas treating apparatus is small as compared to the volume of the closed space, the concentration of the treatment target substances contained in the gas inside the closed space can be efficiently reduced.

The above gas treatment method can be applied to storage chambers.

Namely, the present invention resides in a storage chamber for which the above gas treatment method for treating a gas inside a space is used, the storage chamber including:
a storage space that is the closed space in which a storage target with the treatment target substance adhered thereto is stored; and
the gas treating apparatus placed inside the storage space.

The storage chamber stores sterilized medical tools and the like, for example. As mentioned in PROBLEMS TO BE SOLVED BY THE INVENTION, a small amount of VOCs or treatment target substances inevitably remains on medical tools after the sterilization process, and it is possible that the gas inside the storage chamber contains the substances that have evaporated during storage. The storage chamber according to the present invention causes the decomposition of treatment target substances contained in the gas inside the storage chamber so that the concentration of treatment target substances in the storage chamber is reduced. Accordingly, when an operator opens the door to take out a medical tool stored in the storage chamber, there is less risk of the operator inhaling a gas containing a treatment target substance that may be hazardous to the human body at a concentration of more than a predetermined level.

VOCs as the treatment target substances include acetaldehyde, formaldehyde, ethylene oxide as already mentioned above, as well as phthalaldehyde, glutaraldehyde, and the like.

The above gas treatment method is applicable not only to storage chambers but also to an apparatus in which a gas inside a closed space may contain a treatment target substance.

For example, an incubator is an apparatus in which temperature and humidity are maintained at levels optimal for culturing cells or microbes. Some cells or microbes may release a gas containing a substance classified as a VOC such as acetaldehyde or formaldehyde during growth. In this case, the gas inside the incubator contains VOCs. Namely, similarly to the foregoing, when an operator opens the door of the incubator to take out a culture container, the gas containing VOCs may flow out into the work space and the operator may inhale the gas.

The incubator using the gas treatment method for treating a gas inside a space described above allows decomposition of VOCs contained in the gas inside the incubator even during the culture of cells or microbes so that the concentration of treatment target substances in the incubator is reduced. Accordingly, when an operator opens the door to take out a culture container, there is less risk of the operator inhaling a gas containing a treatment target substance that may be hazardous to the human body at a concentration of more than a predetermined level.

Namely, the present invention resides in an incubator for which the above gas treatment method for treating a gas inside a space is used, the incubator including:
a culture room that is the closed space in which a culture container containing a culture medium that releases the treatment target substance to an outside is stored; and
the gas treating apparatus placed inside the culture room.

A sterilizer is an apparatus that performs a treatment mainly on medical tools to create an environment that prevents the growth of microbes. Specifically, a sterilizing gas such as EOG is introduced into a sealed space in which a medical tool as a sterilization target is accommodated for a predetermined period of time. The apparatus is then exhausted of the gas by aeration. Formaldehyde is also applicable as the sterilizing gas.

However, since ethylene oxide tends to be persistent as mentioned above, even a prolonged time of aeration cannot completely remove ethylene oxide adhered to the medical tool. This issue applies similarly to a case where formaldehyde is adopted as the sterilizing gas. Therefore, when an operator opens the door of the sterilizer to take out a medical tool after the sterilization process, the gas containing VOCs such as ethylene oxide or formaldehyde inside the sterilizer may flow out into the work space and the operator may inhale the gas.

The sterilizer using the gas treatment method for treating a gas inside a space described above allows the decomposition of VOCs contained in the gas inside the sterilizer by means of the gas treating apparatus so that the concentration of VOCs (treatment target substances) in the sterilizer is reduced. Accordingly, when an operator opens the door to take out a medical tool, there is less risk of the operator inhaling a gas containing a treatment target substance that may be hazardous to human body at a concentration of more than a predetermined level.

Namely, the present invention resides in a sterilizer for which the above gas treatment method for treating a gas inside a space is used, the sterilizer including:
a sterilizing space that is the closed space where a sterilization target is placed;
a sterilizing gas inlet that allows a sterilizing gas containing the treatment target substance to be introduced into the sterilizing space; and
the gas treating apparatus placed inside the sterilizing space.

In this configuration, the gas treating apparatus is installed inside the sterilizer. In this case, it is preferable to prevent the sterilizing gas introduced during the sterilization process from adhering to the ozone decomposition filter.

Specifically, the gas treating apparatus may include:
a housing accommodating the excimer lamp;
a gas inlet allowing a gas to be introduced into the housing;
a gas outlet allowing the gas that has been irradiated with the vacuum UV light emitted from the excimer lamp to be discharged out of the housing into the closed space; and
shutters provided near the gas inlet and the gas outlet and configured to allow adjustment of whether or not the gas inside the sterilization space can flow into the housing.

The gas treating apparatus may be disposed of outside the sterilizer and configured to be connected to the sterilizer with a pipe.

Namely, the present invention resides in a sterilizer for which the above gas treatment method for treating a gas inside a space is used, the sterilizer including:
a sterilizing space that is the closed space where a sterilization target is placed;
a sterilizing gas inlet allowing a sterilizing gas containing the treatment target substance to be introduced into the sterilizing space;
the gas treating apparatus placed outside the sterilizing space;
a gas introduction passage connecting a part of the gas treating apparatus and the sterilizing space; and
a gas exhaust passage connecting another part of the gas treating apparatus and the sterilizing space.

In this case, too, it is preferable to prevent the sterilizing gas introduced during the sterilization process from adhering to the ozone decomposition filter. Specifically, the sterilizer may include a valve disposed of in at least one of the gas introduction passage and the gas exhaust passage and configured to be able to let in, stop, and adjust a flow of the gas inside the sterilizing space into the gas treating apparatus.

### EFFECT OF THE INVENTION

According to the present invention, when a gas containing a treatment target substance classified as a VOC exists inside a closed space, the VOC can be treated with higher decomposition efficiency than before.

### BRIEF DESCRIPTION OF THE DRAWINGS

Fig. 1 is a schematic block diagram illustrating a configuration of a storage chamber in a first embodiment of the present invention.
Fig. 2 is a schematic plan view illustrating one example of a gas treating apparatus.
Fig. 3 is a schematic cross-sectional view of an excimer lamp cut in a plane orthogonal to direction d1.
Fig. 4 is a graph showing an emission spectrum of UV light L1 emitted from the excimer lamp enclosed with a light-emitting gas including Xe, overlapped with an oxygen (O₂) absorption spectrum.
Fig. 5 is a schematic cross-sectional view of an excimer lamp in another configuration example cut in a plane orthogonal to direction d1.
Fig. 6 is a schematic cross-sectional view of an excimer lamp in another configuration example cut in a plane orthogonal to direction d1.
Fig. 7 is a schematic plan view illustrating a configuration of an incubator in a second embodiment of the present invention.
Fig. 8A is a schematic plan view illustrating a configuration of a sterilizer in a third embodiment of the present invention.
Fig. 8B is a schematic plan view illustrating the configuration of the sterilizer in the third embodiment of the present invention.
Fig. 8C is a schematic plan view illustrating the configuration of the sterilizer in the third embodiment of the present invention.
Fig. 9A is a schematic plan view illustrating another configuration of the sterilizer in the third embodiment of the present invention.
Fig. 9B is a schematic plan view illustrating another configuration of the sterilizer in the third embodiment of the present invention.
Fig. 10 is a schematic diagram of a storage chamber used in Test 1.
Fig. 11 is a graph showing the results of Test 1.
Fig. 12 is a schematic diagram of an incubator used in Test 2.
Fig. 13 is a graph showing the results of Test 2.

### MODE FOR CARRYING OUT THE INVENTION

Embodiments of a gas treatment method for treating a gas inside a space, a storage chamber, an incubator, and a sterilizer according to the present invention will now be described with reference to the applicable drawings. The drawings referred to below present schematic illustrations and the dimensional ratios in the drawings are not necessarily the same as the actual dimensional ratios. The dimensional ratios in various figures of the drawings are not necessarily the same, either.

### [First embodiment]

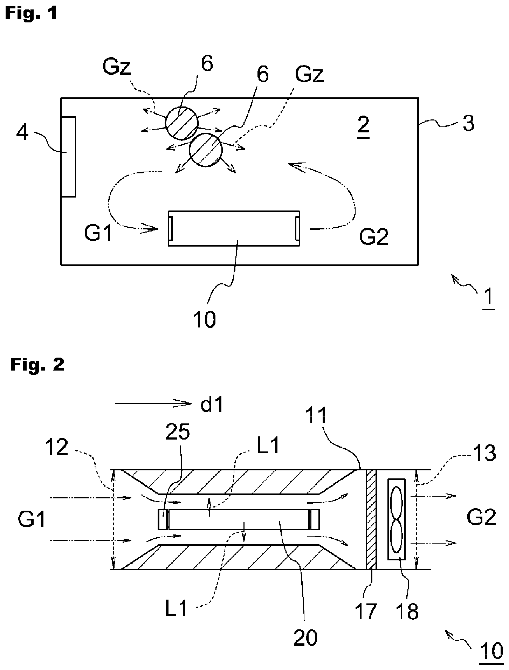

Fig. 1 is a schematic block diagram illustrating a configuration of a storage chamber in a first embodiment of the present invention. The storage chamber 1 is an apparatus having a storage space 2 that is a closed space, for storing storage targets 6 such as medical tools, for example, inside this storage space 2. The storage chamber 1 includes a door 4 which is opened when a storage target 6 is put into the storage space 2, or when a stored storage target 6 is taken out of the storage chamber 1.

The storage chamber 1 includes a gas treating apparatus 10 inside the storage space 2 that is enclosed by outer walls 3. This gas treating apparatus 10 takes in a gas G1 inside the storage space 2, performs a treatment to be described later to this gas G1, and discharges the gas (gas G2) into the same storage space 2.

When a sterilized medical instrument is accommodated inside the storage chamber 1 as a storage target 6, for example, a gas Gz containing a treatment target substance classified as a VOC such as ethylene oxide adhering to the storage target 6 is released into the storage space 2 during storage. When a formalin-fixed sample such as a pathological tissue is stored in the storage chamber 1 as a storage target 6, a gas Gz containing formaldehyde is released from this sample into the storage space 2 during storage in the storage chamber 1. Namely, the gas G1 inside the storage space 2 is composed of air containing such treatment target substances.

Fig. 2 is a schematic plan view illustrating one example of the gas treating apparatus 10. The gas treating apparatus 10 includes a housing 11, an excimer lamp 20 accommodated inside the housing 11, an ozone decomposition filter 17, and a fan 18. The housing 11 includes a gas inlet 12 for introducing a gas G1 from outside the gas treating apparatus 10 into the gas treating apparatus 10, and a gas outlet 13 for discharging a gas G2 to the outside of the gas treating apparatus 10.

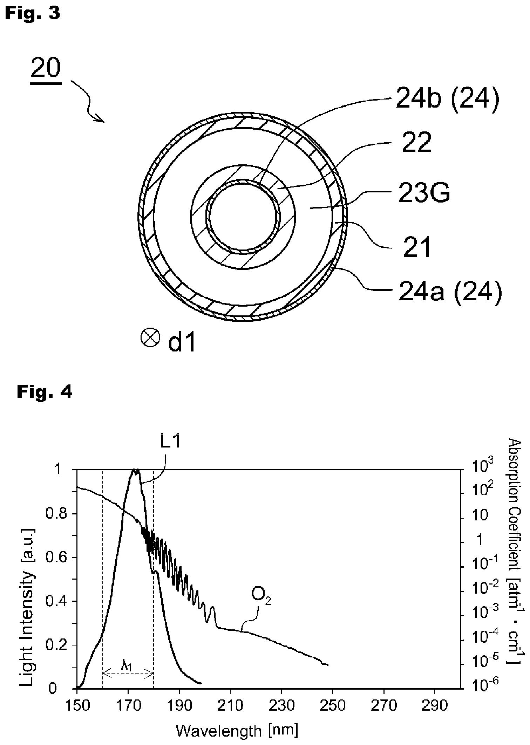

The excimer lamp 20 has a light-emitting tube arranged longitudinally along direction d1 in which the gas G1 flows. Fig. 3 is a schematic cross-sectional view of an excimer lamp 20 cut in a plane orthogonal to direction d1. As shown in Fig. 3, the excimer lamp 20 has light-emitting tubes made up of a tube member that is cylindrical and positioned outside (herein referred to as "outer tube 21") and a tube member disposed inside the outer tube 21, coaxial with the outer tube 21, and having a smaller inside diameter than that of the outer tube 21 (herein referred to as "inner tube 22"). Both light-emitting tubes (21, 22) are made of a dielectric material such as synthetic quartz glass.

The outer tube 21 and inner tube 22 are sealed at the ends in direction d1 (not shown) so that between the tubes there is formed a light-emitting space that is annular when viewed from direction d1. A light-emitting gas 23G that generates excimer molecules by an electric discharge is sealed in this light-emitting space. A more specific example of the light-emitting gas 23G is a gas mixture containing xenon (Xe) and neon (Ne) in a predetermined ratio which may or may not further include a small amount of oxygen or hydrogen.

The excimer lamp 20 of this embodiment includes a first electrode 24a placed on an outer wall surface of the outer tube 21, and a second electrode 24b placed on an inner wall surface of the inner tube 22. The first electrode 24a is in the form of a mesh or a wire. The second electrode 24b is in the form of a film. The second electrode 24b may also be in the form of a mesh or wire similar to the first electrode 24a.

In the example illustrated in Fig. 2, the excimer lamp 20 has a base part 25 in end parts of direction d1 of the light-emitting tubes (21, 22). This base part 25 is made of a ceramic material (inorganic material) such as steatite, forsterite, sialon, alumina, and the like, and serves the function of securing the end of the light-emitting tubes (21, 22). Power supply lines (not shown) are connected to the respective electrodes (24a, 24b) through holes provided to the base parts 25 or via an outer edge of the base part 25.

When a high-frequency AC voltage of about 50 kHz to 5 MHz, for example, is applied between both electrodes (24a, 24b) via the power supply lines from a lamp power source (not shown) of the excimer lamp 20, this voltage is applied to the light-emitting gas 23G via the light-emitting tubes (21, 22). This generates a discharge plasma inside the discharge space filled with the light-emitting gas 23G, and excited atoms of the light-emitting gas 23G form excimers, which, when transiting to the ground state, emit light. In the case where a gas containing xenon (Xe) mentioned above is used as the light-emitting gas 23G, the excimer emits UV light L1 that has a peak wavelength near 172 nm.

The wavelength of the UV light L1 can be changed by using a different substance for the light-emitting gas 23G. For example, ArBr, ArCI, and F2 can be used for the light-emitting gas 23G, which respectively produce vacuum UV light with a peak wavelength near 165 nm, 175 nm, and 153 nm. The description here assumes that the light-emitting gas 23G enclosed in the excimer lamp 20 is a gas containing Xe.

Fig. 4 is a graph showing an emission spectrum of UV light L1 emitted from the excimer lamp 20 enclosed with a light-emitting gas including Xe, overlapped with an oxygen (O₂) absorption spectrum. In Fig. 4, the horizontal axis represents the wavelength, the left vertical axis represents the relative value of light intensity of the excimer lamp, and the right vertical axis represents the absorption coefficient of oxygen(O₂).

When using a gas containing Xe as the light-emitting gas 23G, the UV light L1 emitted from the excimer lamp 20 has a main wavelength within a wavelength band λ₁ of 160 nm or more and less than 180 nm as shown in Fig. 4. As illustrated in Fig. 4, a large amount of light in this wavelength band λ₁ is absorbed by oxygen (O₂).

When the gas treating apparatus 10 starts operating inside the storage chamber 1, the gas G1 inside the storage space 2 is taken into the housing 11 from the gas inlet 12 (this corresponds to step (a)). The gas treating apparatus 10 shown in Fig. 2 is provided with a fan 18 for the purpose of enhancing the flow rate of the gas G1 taken into the gas treating apparatus 10. This fan 18 may be driven and controlled by a power supply unit (not shown) at the same time with power application to the excimer lamp 20.

As mentioned above, the gas G1 is an air containing treatment target substances classified as VOCs such as ethylene oxide released from the storage target 6. This gas G1, while flowing through the gas treating apparatus 10, is irradiated with UV light L1 of the wavelength band λ₁ from the excimer lamp 20. This UV light L1, when absorbed by oxygen (O₂), causes reactions expressed by Formula (1) and Formula (2) set forth in the above section MEANS FOR SOLVING THE PROBLEMS. The formulas are as follows.

O₂+ hv(λ) → O(¹D) + O(³P) ···· (1)

O(¹D) + H₂O → • OH + • OH ···· (2)

Ethylene oxide or EO (herein expressed as "C₂H₄O") is decomposed by oxygen radicals (O(¹D) or O(³P)), or hydroxyl radicals (• OH). Decomposition progresses as expressed by the following formulas, for example, and results in conversion into methanol (CH₃OH) or formaldehyde (HCHO) as intermediate products.

C₂H₄O + • O → • OH + Oxiranyl (C₂H₃O) ···· (3a)

C2H40 + • OH → H₂O + Oxiranyl (C₂H₃O) ···· (3b)

Oxiranyl (C₂H₃O) → CO + • CH₃ ···· (3c)

• CH₃+ O_{2→} CH₃O₂ ···· (3d)

CH₃O₂+ • O → CH₃O • + O₂ ···· (3e)

CH₃O₂+ • OH → CH₃OH + O₂ ···· (3f)

CH₃O • + O₂→ HCHO + H₂O ···· (3g)

Methanol (CH₃OH) produced by the decomposition of ethylene oxide (C₂H₄O) further decomposes into formaldehyde (HCHO) and water by decomposition reactions that progress as follows by hydroxyl radicals (• OH).

CH₃OH + • OH → • CH₂OH + H₂O ···· (4a)

CH₃OH + • OH → CH₃O • + H₂O ···· (4b)

• CH₂OH + O₂→ HCHO + H₂O ···· (4c)

CH₃O • + O₂→ HCHO + H₂O ···· (3g)

Formaldehyde (HCHO) produced by the decomposition of ethylene oxide (C₂H₄O) further decomposes into carbon dioxide and water by decomposition reactions that progress as follows by hydroxyl radicals (• OH).

HCHO + • OH → • CHO + H₂O ···· (5a)

• CHO + O₂→ CO + • HO₂ ···· (5b)

CO + • OH → CO₂+ • H ···· (5c)

Hydrogen radicals (• H) are converted to hydroperoxyl radicals (• HO₂) by following Formula (5d). Hydroperoxyl radicals (• HO₂) are converted to hydroxyl radicals (• OH) by following Formula (5e) and used in the decomposition reactions of VOCs such as ethylene oxide and formaldehyde. Hydroperoxyl radicals (• HO₂) alone are also used in the decomposition reactions of VOCs.

• H + O₂→• HO₂ ···· (5d)

• HO₂+ O₃→• OH + 2O₂ ···· (5e)

Namely, when the gas G1 inside the storage space 2 is irradiated with UV light L1 emitted from the excimer lamp 20, radicals are produced according to above Formula (1) or Formula (2), and these radicals decompose the ethylene oxide contained in the gas G1 (this corresponds to step (b)). As a result, the concentration of ethylene oxide contained in the gas G2 discharged from the gas outlet 13 reduces.

Since the gas treating apparatus 10 discharges the treated gas G2 into the storage space 2, the discharged gas is again introduced into the gas treating apparatus 10 as a gas G1 that is the treatment target. The reactions of the above Formulas (1) to (5c) progress further as the gas is repeatedly irradiated with the UV light L1 so that the concentration of ethylene oxide contained in the gas G1 inside the storage space 2 can be largely reduced.

In the case where the VOC released from the storage target 6 is formaldehyde, the VOC is decomposed in the gas treating apparatus 10 by the reactions of above Formulas (5a) to (5c).

Oxygen atoms O(³P) generated by the reaction of Formula (1) react with oxygen (O₂) contained in the gas G1 and produce ozone (O₃) by Formula (6) set forth in the above section MEANS FOR SOLVING THE PROBLEMS. Formula (6) is as follows.

O(³P) + O₂ → O₃ ···· (6)

Ozone (O₃) is less reactive than radicals and may be left in the gas G2 and discharged from the gas outlet 13 into the storage space 2. Ozone (O₃) is also a substance known to affect the human body. Should the door 4 be opened when the gas G1 inside the storage space 2 still contains ozone (O₃), the operator may inhale the gas G1 containing ozone (O₃).

With this in view, the gas treating apparatus 10 includes an ozone decomposition filter 17 upstream of the gas outlet 13 (closer to the excimer lamp 20) as shown in Fig. 2. Namely, the gas G2 after being irradiated with UV light L1 from the excimer lamp 20 is discharged through the ozone decomposition filter 17 into the storage space 2 (this corresponds to step (c)).

For the ozone decomposition filter 17, for example, structures wherein a catalyst is carried on a base member having a honeycomb structure made of metal such as aluminum and stainless steel, or ceramics, may be employed. For the catalyst, rare metal catalysts such as palladium (Pd), rhodium (Rh), platinum (Pt) and the like, or metal oxides such as manganese dioxide (MnO₂), iron (II) oxide (Fe₂O₃), nickel oxide (NiO) and the like may be used.

According to this configuration, radicals produced by irradiation of UV light L1 contribute to the decomposition reactions of VOCs such as ethylene oxide, while secondary products such as ozone can be removed by the ozone decomposition filter 17.

UV light in the wavelength range of less than 200 nm is largely absorbed by oxygen as shown in Fig. 4. Therefore, if the cross-sectional area of the flow path is too large in a region irradiated with UV light L1, an increased amount of gas G1 may be discharged from the gas treating apparatus 10 without being irradiated with and processed by UV light L1. On the other hand, if openings at the gas inlet 12 and gas outlet 13 have a cross-sectional area that is too small, the flow rate of the gas G1 flowing through the gas treating apparatus 10 will be reduced. In either case, there is a possibility of slowing down the speed of reducing the concentration of VOCs such as ethylene oxide contained in the gas G1 inside the storage space 2.

In this respect, it is preferable to set the cross-sectional area of the flow path in a region irradiated with UV light 1 where the gas G1 flows through smaller than the cross-sectional area of the openings at the gas inlet 12 and gas outlet 13, as in the gas treating apparatus 10 illustrated in Fig. 2. This does not mean that the present invention excludes cases where the cross-sectional areas of the openings at the gas inlet 12 and gas outlet 13 are substantially equal to the cross-sectional area of the flow path in the region irradiated with UV light L1. The same applies to each of the following embodiments.

### (Other configuration examples)

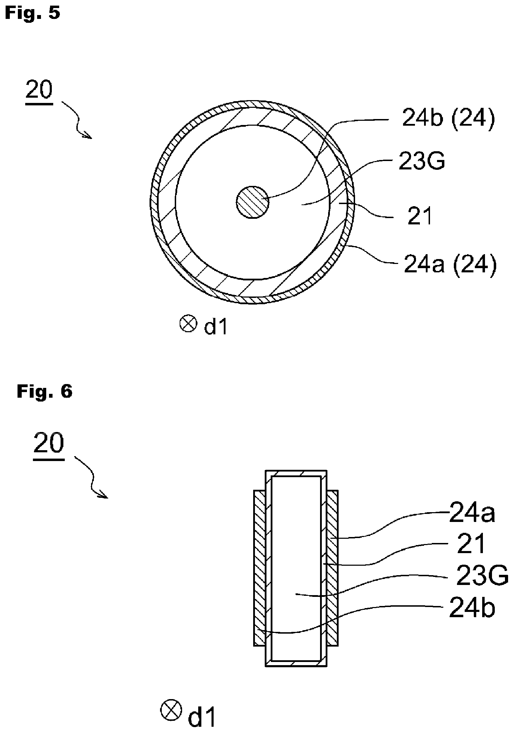

Other configuration examples of the gas treating apparatus 10 are described below. All these configuration examples are also applicable to each of the embodiments described later.
<1> In the example of Fig. 2, one case where the gas treating apparatus 10 includes a fan 18 is described. The gas treating apparatus 10 need not necessarily be equipped with the fan 18 as long as a flow can be generated for causing the gas G1 inside the storage space 2 to be taken into the gas treating apparatus 10.
<2> In the example of Fig. 2, one case where the ozone decomposition filter 17 is mounted inside the housing 11 is described. The ozone decomposition filter 17 need not necessarily be installed in the housing 11 as long as the gas treating apparatus is configured to cause the gas G2 after being irradiated with UV light L1 to pass through the ozone decomposition filter 17. For example, the ozone decomposition filter 17 may be installed outside the gas outlet 13 of the housing 11.
<3> The shape of the excimer lamp 20 is not limited to the one shown in Fig. 3. Structures illustrated in Fig. 5 and Fig. 6 may also be used. Fig. 5 is a schematic cross-sectional view of an excimer lamp 20 having a "single tube structure" cut in a plane orthogonal to direction d1. The excimer lamp 20 illustrated in Fig. 5 has a single light-emitting tube 21 unlike the excimer lamp 20 illustrated in Fig. 3. The light-emitting tube 21 is sealed at the ends of the longitudinal direction, i.e., direction d1 (not shown), and a light-emitting gas 23G is sealed in the interior space. A second electrode 24b is disposed of on the inner side (inside) of the light-emitting tube 21, while the first electrode 24a in the form of a mesh or a wire is disposed of on an outer wall surface of the light-emitting tube 21.

Fig. 6 is a schematic cross-sectional view illustrating an excimer lamp 20 having a "flat-tube structure" and is drawn in accordance with Fig. 3. The excimer lamp 20 illustrated in Fig. 6 has a single light-emitting tube 21 that is rectangular when viewed from the longitudinal direction, i.e., direction d1. The excimer lamp 20 includes a first electrode 24a placed on an outer wall surface on one side of the light-emitting tube 21, and a second electrode 24b placed on an outer wall surface of the light-emitting tube 21 at a position opposite the first electrode 24a. The first electrode 24a and second electrode 24b both have a mesh (reticular) or wire form so as not to inhibit the emission of the UV light L1 generated inside the light-emitting tube 21 out of the light-emitting tube 21.

The cross-sectional shape of the excimer lamp 20 cut in a plane perpendicular to direction d1 is not limited to circular shown in Fig. 3 and Fig. 5 or rectangular shown in Fig. 6. Various shapes can be adopted.

### [Second embodiment]

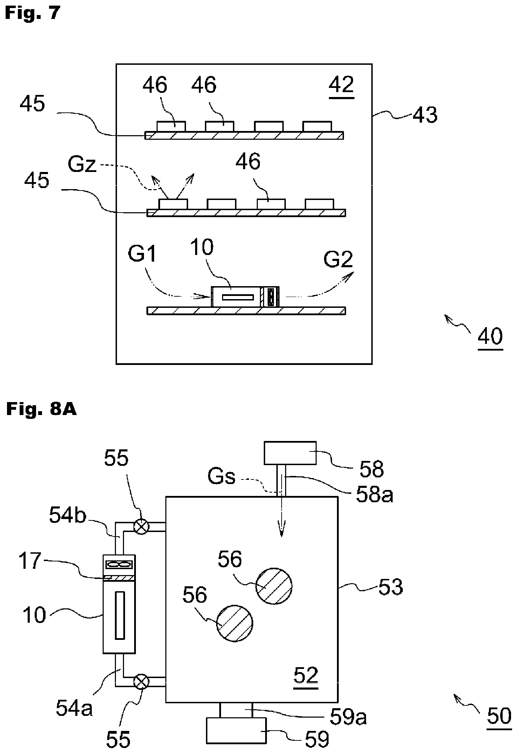

Fig. 7 is a schematic plan view illustrating a configuration of an incubator in a second embodiment of the present invention. The incubator 40 is an apparatus in which temperature and humidity are maintained at levels optimal for culturing cells or microbes, and has a temperature/humidity regulator (not shown). The incubator 40 includes a culture room 42 which is a closed space enclosed by outer walls 43, with shelves 45 inside the culture room 42 for placing culture containers 46. The incubator 40 includes a door (not shown) that is opened when a culture container 46 is placed inside the culture room 42, or when a culture container 46 is taken out of the culture room 42.

The culture container 46 is a container accommodating a culture medium for growing cells or microbes, and made up, for example, of Petri dishes. Some cells or microbes grown in the culture container 46 may release a gas Gz containing treatment target substances classified as VOCs such as acetaldehyde or formaldehyde during growth.

Namely, the gas G1 inside the culture room 42 is composed of air containing such treatment target substances.

The incubator 40 in this embodiment includes a gas treating apparatus 10 similarly to the first embodiment. Therefore, formaldehyde, if contained in the gas Gz released from the culture container 46, is decomposed in the gas treating apparatus 10 by the reactions of above Formulas (5a) to (5e).

In the case where the gas Gz released from the culture container 46 contains acetaldehyde (CH3CHO), reactions progress as expressed by the following formulas, and result in conversion into acetic acid (CH3COOH) or formaldehyde (HCHO) as intermediate products.

CH₃CHO + • OH → CH₃CO • + H₂O ···· (7a)

CH₃CO • + O₂→ CH₃COOO • ···· (7b)

CH₃CO • + O₂→ CO₂+ CH₃O • ···· (7c)

CH₃COOO • + • HO₂→ CH₃COOH + O₃ ···· (7d)

CH₃O • + O₂→ HCHO + • HO₂ ···· (7e)

Acetic acid (CH3COOH) decomposes into carbon dioxide and water by the decomposition reaction that progresses as follows by hydroxyl radicals (• OH).

CH₃COOH + • OH → CO₂+ • CH₃+ H₂O ···· (7f)

Formaldehyde decomposes into carbon dioxide and water by the reactions of Formulas (5a) to (5c) that progress as described in the first embodiment.

Thus, in the incubator 40 of this embodiment, similarly to the storage chamber 1 of the first embodiment, the built-in gas treating apparatus 10 can lower the concentration of acetaldehyde or formaldehyde contained in the gas G1 inside the culture room 42 which is a closed space. Description of other configurations and treatment methods are the same as those of the first embodiment and will be omitted.

When, in particular, formaldehyde released from the culture container 46 exists in the culture room 42 of the incubator 40, the characteristic odor of formaldehyde may become an issue. Since the incubator 40 configured as described above can greatly reduce the concentration of formaldehyde contained in the gas G1 inside the incubator 40, the issue of the odor is also resolved. This issue will be described later with reference to an example.

### [Third embodiment]

Fig. 8A to Fig. 8C are schematic plan views illustrating a configuration of a sterilizer in the third embodiment of the present invention. The sterilizer 50 is an apparatus that performs a sterilization process on target objects such as medical tools to create an environment that prevents the growth of microbes. A specific example may be a medical tool as a storage target 6 stored in the storage chamber 1 of the first embodiment, i.e., a medical tool may be subjected to a sterilization process in the sterilizer 50 of this embodiment, after which it may be taken out of the sterilizer 50 and transferred to a storage chamber.

The sterilizer 50 is an apparatus having a sterilizing space 52 that is a closed space, for storing sterilization targets 56 such as medical tools, for example, in this sterilizing space 52. The sterilizing space 52 is enclosed by outer walls 53. The sterilizer 50 includes a door (not shown) that is opened when a sterilization target 56 is placed inside the sterilizing space 52, or when a sterilization target 56 is taken out after the sterilization process.

The sterilizer 50 includes a sterilizing gas source 58 and a sterilizing gas inlet 58a for introducing a sterilizing gas Gs such as EOG into the sterilizing space 52. The sterilizer 50 also includes an exhaust pump 59 and an exhaust port 59a for drawing the sterilizing gas Gs inside the sterilizing space 52 out of the sterilizer 50 after the sterilization process.

The sterilizer 50 illustrated in Fig. 8A has a gas treating apparatus 10 disposed outside the sterilizing space 52. This gas treating apparatus 10 is in communication with the sterilizing space 52 through pipes (54a, 54b) for gas passage. The pipes (54a, 54b) are provided with valves 55 for controlling the gas flow between the gas treating apparatus and the sterilizing space 52.

During the sterilization process, the sterilizing gas Gs is introduced from the sterilizing gas source 58, with a sterilization target 56 placed inside the sterilizing space 52 as illustrated in Fig. 8A. EOG or gaseous formaldehyde is used as the sterilizing gas Gs. The valves 55 in Fig. 8A at this time are closed so that the sterilizing gas Gs does not flow into the gas treating apparatus 10. This is for preventing the sterilizing gas Gs from adhering to the ozone decomposition filter 17 and compromising the ozone decomposition performance.

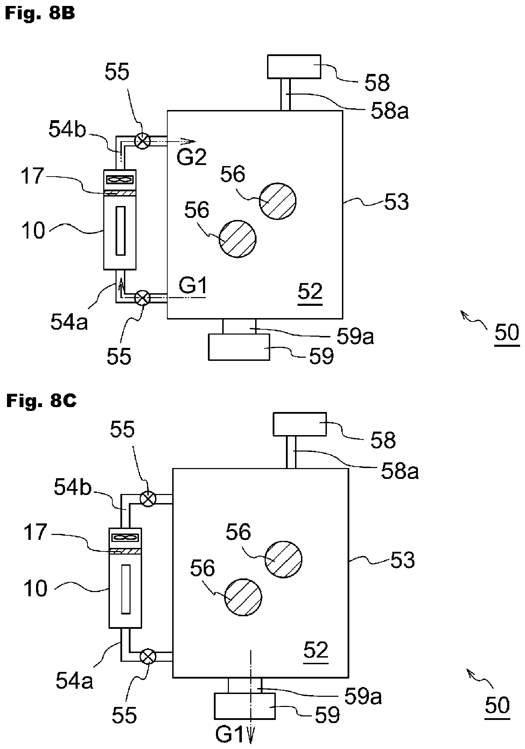

When the sterilization process has finished, there exists a gas G1 that contains a high concentration of VOCs such as ethylene oxide and formaldehyde inside the sterilizing space 52. At this point, the valves 55 are opened as illustrated in Fig. 8B, and the gas treating apparatus 10 is started to operate. This causes the gas G1 inside the sterilizing space 52 to be introduced into the gas treating apparatus 10 through the pipe 54a, where the gas is irradiated with UV light L1 similarly to the first embodiment so that treatment target substances classified as VOCs are decomposed. Gas G2 after the decomposition process is returned into the sterilizing space 52 through the pipe 54b. The pipe 54a corresponds to a "gas introduction passage" and the pipe 54b corresponds to a "gas exhaust passage."

Operating the gas treating apparatus 10 for a predetermined time reduces the concentration of VOCs contained in the gas G1 inside the sterilizing space 52. After the continuous operation of the gas treating apparatus 10 until the concentration reduces to a predetermined reference level, the exhaust pump 59 is started to operate so that the gas G1 inside the sterilizing space 52 is drawn out of the sterilizer 50 from the exhaust port 59a as illustrated in Fig. 8C.

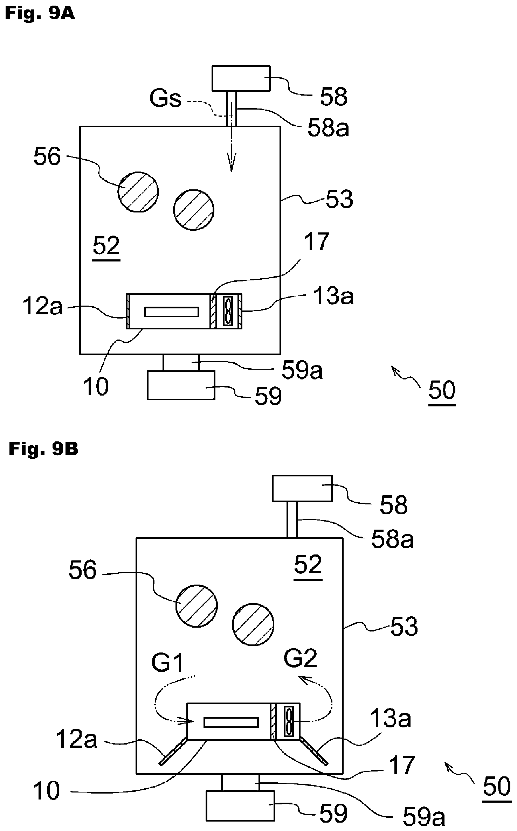

The gas treating apparatus 10 may be disposed of inside the sterilizing space 52 as illustrated in Fig. 9A and Fig. 9B. In this case, as illustrated in Fig. 9B, the gas treating apparatus 10 may include shutters (12a, 13a) that can be opened and closed for controlling the introduction of a gas G1 that is present outside the gas treating apparatus 10 into the gas treating apparatus 10.

Namely, during the sterilizing process when the sterilizing gas Gs is introduced from the sterilizing gas source 58, the shutters (12a, 12b) are closed as illustrated in Fig. 9A. When the sterilizing process is complete, the shutters (12a, 13a) are opened as illustrated in Fig. 9B, and the gas treating apparatus 10 is activated. This way, similarly to the case shown in Fig. 8A, the sterilizing gas Gs is prevented from adhering to the ozone decomposition filter 17 and compromising the ozone decomposition performance.

Description of other configurations and treatment methods are the same as those of the first embodiment and will be omitted.

### [EXAMPLES]

The invention will be described further below with reference to examples.

### (Test 1)

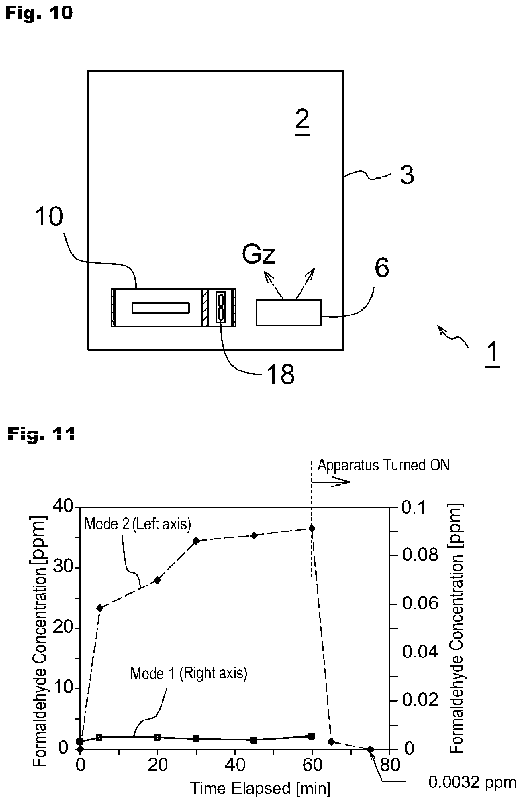

Fig. 10 is a schematic diagram of a storage chamber 1 used in Test 1. The storage chamber 1 has a box-shaped storage space 2 with a volume of 110L, including a gas treating apparatus 10 placed inside. A 90 mm diameter petri dish, into which 10 mL of a 37% formaldehyde solution was dropped, was adopted as the storage target 6.

The concentration of VOCs contained in the gas G1 inside the storage space 2 was measured while operating the gas treating apparatus 10 to determine changes with time. The VOC concentration measurement values are based on readings of an aldehyde sensor ("Tiger" made by RIKEN KEIKI CO., LTD.) placed inside the storage space 2.

In this test, measurements were made to determine changes with time in the concentration of formaldehyde contained in the gas G1 inside the storage space 2 in Mode 1 in which the gas treating apparatus 10 was operated throughout from the measurement start, and in Mode 2 in which the gas treating apparatus 10 was started to operate after 60 minutes had passed from the measurement start. The results are shown in Fig. 11.

During the operation of the gas treating apparatus 10, the fan 18 was driven so that the gas G1 contained inside the storage space 2 was introduced into the gas treating apparatus 10 at a rate of 0.3 m³/min.

The horizontal axis of Fig. 11 represents the time that elapsed from the start of measurement, and the left and right vertical axes both indicate the concentration of formaldehyde contained in the gas G1 inside the storage space 2. The left and right vertical axes show different scales. In Fig. 11, data in Mode 1 is in accordance with the scale of the right vertical axis, and data in Mode 2 is in accordance with the scale of the left vertical axis.

As shown in Fig. 11, it was confirmed that the formaldehyde concentration was far lower than the recommended limit of 0.1 ppm throughout the measurement period from 0 minutes to 60 minutes in Mode 1 in which the gas treating apparatus 10 was operated from the start of measurement.

In Mode 2 in which the gas treating apparatus 10 was started to operate after 60 minutes had passed, the formaldehyde concentration continued to rise until the operation of the gas treating apparatus 10 was started, which confirmed that formaldehyde kept evaporating from the petri dish as the storage target 6. In Mode 2, it was also observed that as soon as the gas treating apparatus 10 was started to operate, the concentration of formaldehyde contained in the gas G1 dropped rapidly. The formaldehyde concentration immediately before the start of operation of the gas treating apparatus 10 was as extremely high as about 35 ppm. After 5 minutes after the gas treating apparatus 10 was started to operate, the formaldehyde concentration dropped to 0.01 ppm or lower.

The test results showed that, with the gas treating apparatus 10 placed inside the storage chamber 1 and operated, a large amount of formaldehyde could be decomposed by a process in a very short time.

### (Test 2)

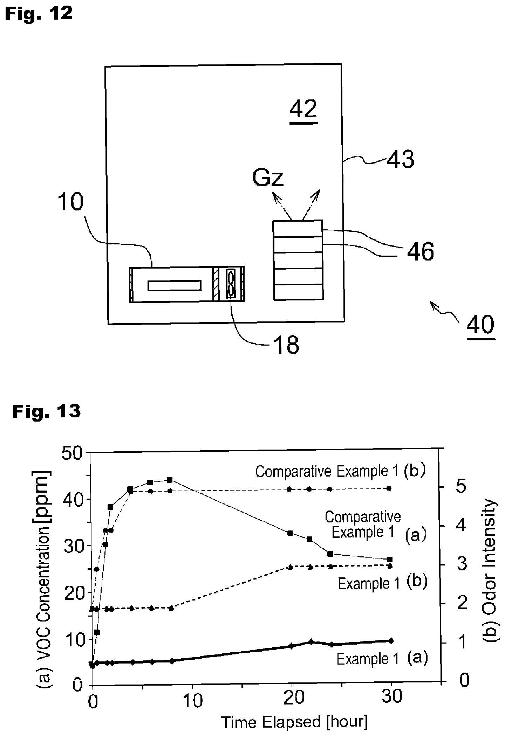

Fig. 12 is a schematic diagram of an incubator 40 used in Test 2. A cool incubator with a culture room 42 having a space volume of 25L (CN-25C made by Mitsubishi Electric Engineering Company, Limited) was employed as the incubator 40. Twenty 90 mm diameter Petri dishes were accommodated inside this culture room 42 as culture containers 46. In ten of the twenty Petri dishes, 0.1 mL of Staphylococcus aureus (NBRC. 12732) was smeared on standard agar (Sample 1). In the remaining ten Petri dishes, 0.1 mL of Escherichia coli (NBRC. 106373) was smeared on standard agar (Sample 2). These twenty Petri dishes correspond to culture containers 46.

In Sample 1, Staphylococcus aureus was added to yield 100 to 300 CFU/Plate. In Sample 2, Escherichia coli was added to yield 100 to 300 CFU/Plate. CFU stands for Colony Forming Unit.

In Example 1, the gas treating apparatus 10 was accommodated inside the incubator 40 with the culture containers 46 (Fig. 12). On the other hand, the gas treating apparatus 10 was not employed in the incubator 40 in Comparative Example 1. In both Example 1 and Comparative Example 1, the VOC concentration and the odor inside the culture room 42 were measured to determine changes in accordance with time from the start of measurement. The results are shown in Fig. 13.

The horizontal axis of Fig. 13 represents the time [hour] that elapsed from the start of measurement, and the left vertical axis (a) represents the concentration of VOC contained in the gas G1 inside the culture room 42. The right vertical axis (b) indicates the intensity of odor inside the culture room 42 on a scale of 0 to 5 (6 levels).

Similar to Test 1, the VOC concentration measurement values are based on readings of an aldehyde sensor placed inside the culture room 42. The odor intensity levels are based on average values of a panel of 3 members in sensory evaluation based on Indication Method of Odor Intensity by 6-point scale. The odor intensity levels 0 to 5 each correspond to the descriptions in the following Table 1.

**[Table 1]**

| Odor intensity | Description |
|---|---|
| 0 | No odor |
| 1 | Odor that can only just be sensed (detection threshold concentration) |
| 2 | Slight odor by which the odor can be identified (recognition threshold concentration) |
| 3 | Odor that can easily be sensed |
| 4 | Strong odor |
| 5 | Intense odor |

Graphs (a) in Fig. 13 show that, in Example 1 which employed the gas treating apparatus 10, the VOC concentration was lower than 10 ppm even after 30 hours had passed from the start of measurement. On the other hand, in Comparative Example 1 which did not employ the gas treating apparatus 10, the VOC concentration rose rapidly from the start of measurement, and reached about 45 ppm after 10 hours. The VOC concentration then reduced with time but remained over 20 ppm.

Graph (a) of Comparative Example 1 shows a slight reduction in VOC concentration after the elapse of 10 hours. This is assumed to be attributed to the slowing down of microbial growth and leaking of a slight amount of gas through minute gaps present in the incubator 40.

Graphs (b) in Fig. 13 show that the odor inside the incubator 40 of Comparative Example 1 intensified to reach level 5 of odor intensity within 5 hours from the start of measurement and stayed at this intensity level even after the elapse of 30 hours. In contrast, while the odor intensity increased with time in the incubator 40 of Example 1, the odor intensity remained at level 3 even after the elapse of 30 hours.

These test results confirmed the VOC decomposition performance of the incubator 40 according to the present invention whereby VOCs contained in the gas G1 inside the culture room 42 are decomposed. It was also confirmed that this decomposition process provides the effect of suppressing odor inside the culture room 42.

Table 2 shows the results of measurements of numbers of bacterial colonies contained in each of the samples of both Example 1 and Comparative Example 1 after 30 hours passed. The values are all average values of numbers of bacteria that exist on three Petri dishes.

**[Table 2]**

| | Example 1 (CFU/Plate) | Comparative Example 1 (CFU/Plate) |
|---|---|---|
| Staphylococcus aureus | 193 | 195 |
| Escherichia coli | 153 | 158 |

Table 2 shows that there is no significant difference in the numbers of colonies of both bacteria after the elapse of 30 hours between Example 1 which employed the gas treating apparatus 10 and Comparative Example 1 which did not employ the gas treating apparatus 10. This leads to a conclusion that the exhaust gas G2 from the gas treating apparatus 10 discharged into the culture room 42 does not adversely affect the cells being grown.

### [Other Embodiments]

The storage chamber 1 described in the first embodiment may have any configuration as long as a storage target 6 that releases a gas containing a treatment target substance classified as a VOC is stored inside the storage space 2 which is a closed space. For example, in a case where a formalin-fixed sample such as a pathological tissue is transported in an automobile, either placed inside a cab sharing the same space as the driver's seat, or inside a trunk of an automobile in the form of a station wagon in which the trunk is connected to the passenger room, the cab or passenger room of such automobile corresponds to the storage chamber 1.

### DESCRIPTION OF REFERENCE SIGNS

- 1: Storage chamber
- 2: Storage space
- 3: Outer wall
- 4: Door
- 6: Storage target
- 10: Gas treating apparatus
- 11: Housing
- 12: Gas inlet
- 12a: Shutter
- 13: Gas outlet
- 13a: Shutter
- 17: Ozone decomposition filter
- 18: Fan
- 20: Excimer lamp
- 21: Light-emitting tube (outer tube)
- 22: Light-emitting tube (inner tube)
- 23G: Light-emitting gas
- 24a: First electrode
- 24b: Second electrode
- 25: Base part
- 40: Incubator
- 42: Culture room
- 43: Outer wall
- 45: Shelf
- 46: Culture container
- 50: Sterilizer
- 52: Sterilizing space
- 53: Outer wall
- 54a: Pipe
- 54b: Pipe
- 55: Valve
- 56: Sterilization target
- 58: Sterilizing gas source
- 58a: Sterilizing gas inlet
- 59: Exhaust pump
- 59a: Exhaust port

## Claims

1. A gas treatment method for treating a gas inside a space, the gas being an air containing a treatment target substance classified as a VOC and existing inside a closed space, the method comprising the steps of:
(a) drawing the gas inside the closed space into a gas treating apparatus including an excimer lamp capable of emitting vacuum UV light;
(b) producing radicals and decomposing the treatment target substance by irradiating the gas inside the gas treating apparatus with the vacuum UV light from the excimer lamp; and
(c) discharging the gas after a treatment subjected to the step (b) via an ozone decomposition filter into the closed space.

2. The gas treatment method for treating a gas inside a space according to claim 1, wherein the gas treating apparatus includes:
a housing accommodating the excimer lamp;
a gas inlet allowing the gas to be introduced into the housing;
a gas outlet allowing the gas that has been irradiated with the vacuum UV light emitted from the excimer lamp to be discharged out of the housing into the closed space; and
the ozone decomposition filter disposed of inside the housing between the excimer lamp and the gas outlet.

3. The gas treatment method for treating a gas inside a space according to claim 2, wherein the housing has a smaller cross-sectional area of a flow path in a space irradiated with the vacuum UV light than a cross-sectional area of an opening at the gas inlet.

4. The gas treatment method for treating a gas inside a space according to any one of claims 1 to 3, wherein the gas treating apparatus includes a fan, and a power supply unit that drives the fan and the excimer lamp,
the step (a) being a step of drawing the gas inside the closed space into the gas treating apparatus by the fan, and
the steps (a) to (c) being repeatedly performed in which the gas discharged from the gas treating apparatus into the closed space by the step (c) is taken into the closed space again by the step (a).

5. The gas treatment method for treating a gas inside a space according to any one of claims 1 to 4, wherein the treatment target substance is a substance containing at least one of the group consisting of ethylene oxide and aldehydes.

6. The gas treatment method for treating a gas inside a space according to any one of claims 1 to 5, wherein the excimer lamp includes a light-emitting tube enclosed with a light-emitting gas containing Xe.

7. The gas treatment method for treating a gas inside a space according to any one of claims 1 to 6, wherein the gas treating apparatus is placed at an installation position inside the closed space, and
the steps (a) to (c) are performed while the installation position of the gas treating apparatus inside the closed space is changed.

8. A storage chamber for which the gas treatment method for treating a gas inside a space according to any one of claims 1 to 7 is used, the storage chamber comprising:
a storage space that is the closed space in which a storage target with the treatment target substance adhered thereto is stored; and
the gas treating apparatus placed inside the storage space.

9. An incubator for which the gas treatment method for treating a gas inside a space according to any one of claims 1 to 7 is used, the incubator comprising:
a culture room that is the closed space in which a culture container containing a culture medium that releases the treatment target substance to an outside is stored; and
the gas treating apparatus placed inside the culture room.

10. A sterilizer for which the gas treatment method for treating a gas inside a space according to any one of claims 1 to 6 is used, the sterilizer comprising:
a sterilizing space that is the closed space where a sterilization target is placed;
a sterilizing gas inlet allowing a sterilizing gas containing the treatment target substance to be introduced into the sterilizing space;
the gas treating apparatus placed outside the sterilizing space;
a gas introduction passage connecting a part of the gas treating apparatus and the sterilizing space; and
a gas exhaust passage connecting another part of the gas treating apparatus and the sterilizing space.

11. The sterilizer according to claim 10, further comprising a valve disposed of in at least one of the gas introduction passage and the gas exhaust passage and configured to allow adjustment of whether or not the gas inside the sterilization space can flow into the gas treating apparatus.

12. A sterilizer for which the gas treatment method for treating a gas inside a space according to any one of claims 1 to 7 is used, the sterilizer comprising:
a sterilizing space that is the closed space where a sterilization target is placed;
a sterilizing gas inlet allowing a sterilizing gas containing the treatment target substance to be introduced into the sterilizing space; and
the gas treating apparatus placed inside the sterilizing space.

13. The sterilizer according to claim 12, wherein the gas treating apparatus includes:
a housing accommodating the excimer lamp;
a gas inlet allowing a gas to be introduced into the housing;
a gas outlet allowing the gas that has been irradiated with the vacuum UV light emitted from the excimer lamp to be discharged out of the housing into the closed space; and
shutters provided near the gas inlet and the gas outlet and configured to allow adjustment of whether or not the gas inside the sterilization space can flow into the housing.
